# EUROPÄISCHE PATENTANMELDUNG

(11) **EP 2 371 265 A2**
(43) Veröffentlichungstag der Anmeldung: **05.10.2011**
(21) Anmeldenummer: 11159315.8
(22) Anmeldetag: 23.03.2011
(51) Int. Cl.: A61B 1/00, A61B 1/07, A61B 1/06, G02B 23/24

(54) **Vorrichtung zur Bereitstellung von weißem Beleuchtungslicht**

(30) Priorität: 29.03.2010 DE 102010013308
(71) Anmelder: Karl Storz GmbH & Co. KG, 78532 Tuttlingen (DE)
(72) Erfinder: Ehrhardt, André, Dr., 78532, Tuttlingen (DE); Irion, Klaus M., 78532, Tuttlingen (DE); Fallert, Johannes, 78532, Tuttlingen (DE); Schwarz, Peter, 78532, Tuttlingen (DE)

(57) **Zusammenfassung**

Eine Vorrichtung (20) zur Bereitstellung von weißem Beleuchtungslicht für medizinische oder boroskopische Anwendungen umfasst eine erste Lichtquelle (21) mit einer Leuchtdiode, zur Emission von Licht mit einem breiten Spektrum (47), eine zweite Lichtquelle (22) zur Emission von monochromatischem Licht, und eine Einkopplungseinrichtung (25) zum Einkoppeln von Licht der ersten Lichtquelle (21) und Licht der zweiten Lichtquelle (22) in einen gemeinsamen Strahlengang (27), um Beleuchtungslicht mit einer verbesserten Farbwiedergabe zu erzeugen.

## Beschreibung

Die vorliegende Erfindung bezieht sich auf eine Vorrichtung und ein Verfahren zur Bereitstellung von weißem Beleuchtungslicht, insbesondere für endoskopische oder exoskopische Anwendungen.

Das Beleuchtungslicht zur Beleuchtung von Objekten bei endoskopischen, exoskopischen oder mikroskopischen Untersuchung wurde lange Zeit fast ausschließlich mittels Glühlampen und verschiedener Typen von Gasentladungslampen erzeugt. Glühlampen erzeugen näherungsweise Plancksche Spektren mit geringer Struktur. Die Spektren weisen eine relativ geringe Farbtemperatur auf, werden vom menschlichen Auge aber als weiß wahrgenommen. Aufgrund der Ähnlichkeit der Spektren von Glühlampen mit dem idealen Planckschen Spektrum ist auch die Farbwiedergabe sehr gut.

Auch bei Beleuchtung mittels hochwertiger Gasentladungslampen kann eine gute bis sehr gute Farbwiedergabe erzielt werden. Gleichzeitig bieten einige hochwertige Typen von Gasentladungslampen höhere Farbtemperaturen, deutlich höherer Lichtausbeute und eine höhere Leistungsdichte als Glühlampen.

Bei vielen Anwendungen, bei denen Farbwiedergabe eine geringere Rolle spielt, werden zunehmend Weißlicht-Leuchtdioden eingesetzt. Weißlicht-Leuchtdioden weisen Lichtausbeuten auf, die teilweise im Bereich der Wirkungsgrade von hochwertigen Gasentladungslampen oder darüber liegen und damit ebenfalls deutlich höher sind als Wirkungsgrade von Glühlampen. Vorteile vieler Weißlicht-Leuchtdioden bestehen in einer deutlich einfacheren elektrischen Beschaltung bzw. Leistungsversorgung, vergleichsweise geringen Herstellungskosten und vergleichsweise sehr langen Lebensdauern.

In der EP 1 894 516 A1 ist ein Beleuchtungssystem zum Erzeugen von Licht mittels einer Leuchtdiode beschrieben. Das Licht der Leuchtdiode wird über ein Lichtleitkabel zu einem Endoskop oder zu einem Mikroskop übertragen.

Die beiden wichtigsten Typen von Weißlicht-Leuchtdioden beruhen auf einer Kombination von zwei oder drei einfarbigen Leuchtdioden bzw. Halbleiterübergängen (rot, grün, blau-RGB) und auf einer teilweisen Verschiebung des Lichts einer einzelnen blauen Leuchtdiode durch fluoreszierende oder phosphoreszierende Farbstoffe in grüne, gelbe und rote Wellenlängenbereiche. Beiden Konzepten ist jedoch eine vergleichsweise schlechte Farbwiedergabe gemein. Diese beruht auf den zerklüfteten Emissionsspektren, die oft von wenigen ausgeprägten Maxima und ebenso ausgeprägten Minima geprägt werden.

Die Qualität der Farbwiedergabe ist jedoch bei vielen Anwendungen von herausragender Bedeutung. Beispielsweise in der medizinischen Diagnostik ermöglicht nur eine Lichtquelle, die eine sehr gute Farbwiedergabe bietet, eine Wahrnehmung geringer Farbunterschiede, die beispielsweise auf krankhafte Gewebeveränderungen hinweisen können. Auch außerhalb der Medizin können manche chemischen oder strukturellen Eigenschaften einer Oberfläche oder ihr Gefüge sowie Veränderungen umso besser beobachtet, identifiziert und unterschieden werden, je besser die Farbwiedergabe ist.

Eine Aufgabe der vorliegenden Erfindung besteht darin, eine verbesserte Vorrichtung und ein verbessertes Verfahren zum Bereitstellen von weißem Beleuchtungslicht für medizinische Anwendungen zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, zur Erzeugung von weißem Beleuchtungslicht für medizinische Anwendungen das Licht einer Leuchtdiode, insbesondere einer Weißlicht-Leuchtdiode, durch das monochromatische Licht einer zweiten Lichtquelle zu ergänzen, um Beleuchtungslicht mit einem verbesserten Spektrum, insbesondere mit einer verbesserten Farbwiedergabe, zu erzeugen. Die Qualität der Farbwiedergabe wird beispielsweise anhand eines definierten Farbwiedergabeindex oder anhand des bei Beleuchtung eines vorbestimmten Satzes von Testfarben vorliegenden mittleren Farbabstands bestimmt. Bei Verwendung einer bestimmten Kamera kann auch die Abweichung der bei einem Weißabgleich ermittelten Weißabgleichparameter vom Idealwert 1 als Maß für die Güte der Farbwiedergabe verwendet werden.

Eine Vorrichtung zur Bereitstellung von weißem Beleuchtungslicht für medizinische Anwendungen umfasst eine erste Lichtquelle mit einer Leuchtdiode, zur Emission von Licht mit einem breiten Spektrum; eine zweite Lichtquelle zur Emission von monochromatischem Licht; und eine Einkopplungseinrichtung zum Einkoppeln von Licht der ersten Lichtquelle und Licht der zweiten Lichtquelle in einen gemeinsamen Strahlengang, um Beleuchtungslicht mit einer verbesserten Farbwiedergabe zu erzeugen.

Die Vorrichtung ist insbesondere ein Lichtquellenvorrichtung. Die Vorrichtung ist insbesondere zur Bereitstellung von weißem Beleuchtungslicht für endoskopische, exoskopische, mikroskopische und andere medizinische Anwendungen ausgebildet. Alternativ oder gleichzeitig ist die Vorrichtung zur Bereitstellung von weißem Beleuchtungslicht für boroskopische bzw. nicht-medizinisch endoskopische Anwendungen ausgebildet.

Ein Exoskop ist eine zum extrakorporalen Gebrauch vorgesehene und ausgebildete Vorrichtung zur visuellen Inspektion bzw. Betrachtung von Objekten in der Medizin, insbesondere von Objekten an oder nahe äußeren Oberflächen eines menschlichen oder tierischen Körpers. Im Unterschied zu einem Endoskop ist ein Exoskop nicht dazu ausgebildet, um durch eine kleine natürliche oder künstliche Öffnung in einen natürlichen oder künstlichen Hohlraum eingeführt zu werden. Ein Exoskop ist vielmehr für eine Betrachtung eines Objekts ausgebildet, das zumindest während der Betrachtung, insbesondere während einer Operation, von außen sichtbar ist. Entsprechend befindet sich das Exoskop während seines bestimmungsgemäßen Einsatzes vollständig außerhalb des menschlichen oder tierischen Körpers und weist im Unterschied zum Endoskop nicht unbedingt einen langen dünnen Schaft auf.

Ein Exoskop kann ein oder zwei Kameras oder lichtempfindliche Bildsensoren zur zweidimensionalen oder dreidimensionalen Erfassung und Darstellung, beispielsweise auf einem Bildschirm, aufweisen. Alternativ ist ein Exoskop monokular oder binokular für die unmittelbare Betrachtung mit dem menschlichen Auge ausgebildet. Ein Exoskop ist in der Regel für eine Gegenstandsweite im Bereich von einigen oder wenigen Zentimetern oder wenigen Dezimetern ausgebildet oder optimiert. Ein Exoskop kann eine starke Vergrößerung aufweisen, die eine Auflösung ermöglicht, die mit bloßem Auge nicht erreichbar ist, und damit Eigenschaften einer Lupe bzw. Stereolupe oder eines Mikroskops bzw. Stereomikroskops aufweisen. Vom Mikroskop bzw. Stereomikroskop unterscheidet sich das Exoskop in der Regel durch eine größere Gegenstandsweite.

Die Leuchtdiode der ersten Lichtquelle kann einen anorganischen oder einen organischen Halbleiter umfassen. Die Leuchtdiode ist insbesondere eine Weißlicht-Leuchtdiode. Wie bereits eingangs erwähnt, umfassen Weißlicht-Leuchtdioden beispielsweise drei Halbleiterübergänge, von denen jeweils einer Licht im blauen, grünen bzw. roten Spektralbereich emittiert. Diese drei oder mehr einzelnen, jeweils ein schmalbandiges Spektrum emittierenden Halbleiterübergänge können in einem Array angeordnet sein, beispielsweise in einer Reihe bzw. Zeile mit drei einzelnen Halbleiterübergängen oder in zwei Reihen bzw. Zeilen mit jeweils zwei oder drei einzelnen Halbleiterübergängen. Die Spektren und die Strahlungsleistungen der einzelnen Halbleiterübergänge sind so auf einander abgestimmt, dass das von allen Halbleiterübergängen zusammen erzeugte Licht vom menschlichen Auge als weiß oder im Wesentlichen weiß wahrgenommen wird.

Vom menschlichen Auge als weiß empfunden wird Licht insbesondere, wenn ihm eine Farbtemperatur zwischen 3500 Kelvin und 6500 Kelvin, manchmal auch schon ab 2500 Kelvin zugeordnet werden kann, und wenn der Farbwiedergabeindex nicht zu niedrig ist, insbesondere mindestens 50, 70 oder 80 beträgt.

Eine weiterer verbreiteter Typ Weißlicht-Leuchtdiode umfasst lediglich einen Halbleiterübergang, der Licht im blauen Spektralbereich emittiert, und einen fluoreszierenden oder phosphoreszierenden Farbstoff, der das blaue Licht teilweise in grünes, gelbes und/oder rotes Licht wandelt.

Die zweite Lichtquelle ist insbesondere zur Emission von monochromatischem Licht im grünen Spektralbereich (Wellenlängen von ca. 520 nm bis ca. 565 nm), im gelben Spektralbereich (ca. 565 nm bis 575 nm) oder im roten Spektralbereich (ab ca. 600 nm) ausgebildet.

Die erste Lichtquelle ist zur Emission von Licht mit einem breiten Spektrum ausgebildet. Dies bedeutet, dass das von der ersten Lichtquelle emittierte Licht nicht monochromatisch ist sondern viele Wellenlängen bzw. ein breites Kontinuum von Wellenlängen umfasst. Das Spektrum des Lichts der ersten Lichtquelle weist eine Breite von mehreren zehn nm oder sogar von mehreren hundert nm auf.

Die zweite Lichtquelle ist zur Emission von monochromatischem Licht ausgebildet. Dies bedeutet, dass das von der zweiten Lichtquelle emittierte Licht nur eine oder wenige Wellenlängen umfasst und sein Spektrum eine Breite von wenigen nm oder weniger aufweist oder aus einer oder mehreren schmalen Linien besteht. Ein Beispiel ist ein Spektrum mit einer oder mehreren schmalen Linien, wie es von vielen Lasern zumindest im kontinuierlichen Betrieb (cw) erzeugt wird.

Die Breite eines Spektrums ist die Größe des Wellenlängenbereichs, innerhalb dessen ein vorbestimmter Anteil (beispielsweise 70 %, 80 % oder 90 %) der gesamten im für das menschliche Auge sichtbaren Spektralbereich (380 nm bis 780 nm) erzeugten Strahlungsleistung liegt, wobei der Wellenlängenbereich insofern symmetrisch ist, als die spektrale Strahlungsleistung an der unteren und an der oberen Grenze des Wellenlängenbereichs den gleichen Wert aufweist.

Beim Mischen von Licht der ersten Lichtquelle und Licht der zweiten Lichtquelle wird gleichzeitig Licht der ersten Lichtquelle und Licht der zweiten Lichtquelle in den selben Beleuchtungs-Strahlengang eingekoppelt und über diesen für eine endoskopische, exoskopische oder mikroskopische medizinische oder boroskopische Anwendung bereitgestellt. Im Beleuchtungs-Strahlengang kann sich die Intensität oder die Strahlungsleistung des von der ersten Lichtquelle erzeugten Lichts von der Intensität bzw. Strahlungsleistung des von der zweiten Lichtquelle erzeugten Lichts deutlich unterscheiden. Im Beleuchtungs-Strahlengang kann die Strahlungsleistung des von der zweiten Lichtquelle erzeugten Lichts deutlich geringer als die Strahlungsleistung des von der ersten Lichtquelle erzeugten Lichts sein. Gleichzeitig kann die spektrale Strahlungsleistung des von der zweiten Lichtquelle erzeugten Lichts in einem kleinen Wellenlängenbereich größer als die von der ersten Lichtquelle erzeugte Strahlungsleistung sein. Innerhalb eines Querschnitts des Beleuchtungs-Strahlengangs können sich die Ortsabhängigkeiten der Intensitäten oder Strahlungsleistungen des von der ersten Lichtquelle erzeugten Lichts und des von der zweiten Lichtquelle erzeugten Lichts unterscheiden.

Ein Vorteil der Vorrichtung besteht darin, dass die Farbwiedergabe des Beleuchtungslichts nicht subtraktiv mittels eines Filters, sondern weitgehend oder vollständig additiv verbessert werden kann. Ein Filter mindert die spektrale Strahlungsleistung und die spektrale Intensität in einem bestimmten Spektralbereich und damit auch die über den gesamten für das menschliche Auge sichtbaren Spektralbereich integrierte Strahlungsleistung bzw. Intensität. Dies ist besonders nachteilig bei der Einkopplung in dünne bzw. dünnkalibrige Lichtwellenleiter oder Lichtleitkabel, wie sie in der Endoskopie zur Übertragung des Beleuchtungslichts zum distalen Ende des Endoskops verwendet werden.

Im Gegensatz dazu kann die hier beschriebene Vorrichtung Beleuchtungslicht mit einer verbesserten Farbwiedergabe bereitstellen, indem dem Licht der ersten Lichtquelle Licht der zweiten Lichtquelle hinzugefügt wird bzw. das Licht der ersten Lichtquelle und das Licht der zweiten Lichtquelle gemischt und gleichzeitig in einen gemeinsamen Strahlengang eingekoppelt werden. Abhängig von der Ausbildung der Einkopplungseinrichtung kann dies ohne Verlust oder bei lediglich geringem Verlust von Strahlungsleistung der ersten Lichtquelle realisiert werden. Die Strahlungsleistung der Vorrichtung kann dadurch gegenüber der ersten Lichtquelle allein sogar noch erhöht sein. Mittels einfacher Berechnungen oder Simulationen kann für Vorrichtungen, wie sie hier beschrieben sind, abgeschätzt werden, dass Verbesserungen der gesamten in den Beleuchtungs-Strahlengang eingekoppelten Strahlungsleistung im für das menschliche Auge sichtbaren Spektralbereich von 20 % bis 40 % möglich sind. Dies ist besonders in Hinblick darauf vorteilhaft, dass Leuchtdioden in vielen Fällen eine geringere Flächendichte der Strahlungsleistung aufweisen als Glühlampen oder Gasentladungslampen.

Bei einer Vorrichtung, wie sie hier beschrieben ist, ist die Einkopplungseinrichtung insbesondere so ausgebildet, dass am Ort der Einkopplung der Querschnitt des von der zweite Lichtquelle ausgehenden Lichtbündels kleiner ist als der Querschnitt des von der ersten Lichtquelle ausgehenden Lichtbündels. Dazu umfasst die Einkopplungseinrichtung beispielsweise einen Lichtwellenleiter mit einem ersten Ende und einem zweiten Ende, wobei das erste Ende des Lichtwellenleiters mit der zweiten Lichtquelle gekoppelt ist, und wobei das zweite Ende des Lichtwellenleiters neben, in oder lichtstromabwärts vor der lichtemittierenden Fläche der ersten Lichtquelle angeordnet ist. Die erste Lichtquelle kann in einem gemeinsamen Gehäuse oder einer gemeinsamen Baugruppe mit der zweiten Lichtquelle angeordnet sein. Alternativ ist die erste Lichtquelle beispielsweise am proximalen Ende oder am distalen Ende eines Endoskops angeordnet, wobei die zweite Lichtquelle über ein Lichtleitkabel mit dem Endoskop gekoppelt sein kann.

Die lichtemittierende Fläche der ersten Lichtquelle ist insbesondere größer oder wesentlich größer als die Querschnittsfläche einer Durchgangsbohrung in der ersten Lichtquelle, in der das zweite Ende des Lichtwellenleiters angeordnet ist. Das Verhältnis zwischen der lichtemittierenden Fläche der ersten Lichtquelle und der Querschnittsfläche der Durchgangsbohrung beträgt beispielsweise mindestens 2:1, 5:1, 10:1, 20:1, 50:1 oder 100:1. Beispielsweise ist die lichtemittierende Fläche der Leuchtdiode quadratisch oder rechteckig mit einer Seitenlänge zwischen 1 mm und 3 mm, insbesondere mit einer Seitenlänge von ca. 2 mm. Die Durchgangsbohrung kann einen Durchmesser von 100 µm oder weniger, im Einzelfall auch von mehreren hundert µm aufweisen. Die lichtemittierende Fläche ist damit mindestens ca. 100 mal so groß wie die Querschnittsfläche der Durchgangsbohrung.

Ein Vorteil der Verwendung eines Lichtwellenleiters zur Einkopplung des von der zweiten Lichtquelle ausgehenden zweiten Lichtbündels in den Beleuchtungsstrahlengang besteht darin, dass der Lichtwellenleiter mit seinem geringen Querschnitt nur eine kleine Öffnung in der lichtemittierenden Fläche der ersten Lichtquelle benötigt bzw. das Licht der ersten Lichtquelle nur in geringem Maße abschattet bzw. eine Einkopplung des Lichts der zweiten Lichtquelle unmittelbar am Rand der lichtemittierenden Fläche der ersten Lichtquelle ermöglicht. Soweit das Licht der zweiten Lichtquelle verlustarm in die Lichtwellenleiter eingekoppelt werden kann, ermöglicht die Lichtquellenvorrichtung eine effiziente Einkopplung des Lichts der ersten Lichtquelle und des Lichts der zweiten Lichtquelle in den gemeinsamen Strahlengang.

Bei einer Lichtquellenvorrichtung, wie sie hier beschrieben ist, kann die Einkopplungseinrichtung zumindest entweder ein Objektiv, einen gekrümmten Spiegel, ein optisches Gitter oder eine andere abbildende Einrichtung umfassen, welche den Querschnitt des von der zweiten Lichtquelle erzeugten Lichtbündels verringert. Ein Objektiv umfasst eine oder mehrere Linsen und/oder Linsengruppen und/oder einen oder mehrere gekrümmte Spiegel und wirkt insbesondere sammelnd. Die abbildende Einrichtung kann ausgebildet und angeordnet sein, um am Rand der lichtemittierenden Fläche der ersten Lichtquelle oder in einer Öffnung in der lichtemittierenden Fläche der ersten Lichtquelle oder im Beleuchtungsstrahlengang vor der lichtemittierenden Fläche der ersten Lichtquelle eine Taille bzw. eine Einschnürung bzw. eine Engstelle des von der zweiten Lichtquelle ausgehenden Lichtbündels zu erzeugen. Insbesondere erzeugt die abbildende Einrichtung eine Taille am Ort der Einkopplung.

Dabei kann die Einkopplungseinrichtung ferner einen Umlenkspiegel umfassen, der im Beleuchtungsstrahlengang lichtstromabwärts vor der lichtemittierenden Fläche der ersten Lichtquelle angeordnet ist, und an dem die von der abbildenden Einrichtung erzeugte Taille des von der zweiten Lichtquelle erzeugten Lichtbündels liegt. Der Umlenkspiegel bzw. seine reflektierende Fläche bildet den Ort der Einkopplung, ab dem das zweite Lichtbündel im Beleuchtungsstrahlengang und im Wesentlichen in die gleiche Richtung wie das erste Lichtbündel läuft.

Der Umlenkspiegel ist kleiner oder wesentlicher kleiner als der Querschnitt des von der ersten Lichtquelle ausgehenden Lichtbündels am Ort des Umlenkspiegels, um einen möglichst geringen Teil des ersten Lichtbündels abzuschatten. Beispielsweise beträgt die auf eine Ebene senkrecht zur Hauptausbreitungsrichtung des von der ersten Lichtquelle ausgehenden Lichtbündels projizierte Fläche des Umlenkspiegels höchstens ein Zehntel, ein Fünftel oder die Hälfte der Querschnittsfläche des von der ersten Lichtquelle erzeugten Lichtbündels am Ort des Umlenkspiegels. Die Querschnittsfläche des von der ersten Lichtquelle ausgehende Lichtbündels wird dabei insbesondere in einer Ebene senkrecht zur Hauptausbreitungsrichtung des Lichtbündels gemessen, die den Mittelpunkt, insbesondere den Flächenschwerpunkt, des Umlenkspiegels enthält.

Eine derartige Taillierung bzw. Einschnürung des zweiten Lichtbündels am Ort der Einkopplung ermöglicht eine besonders geringe Störung des von der ersten Lichtquelle erzeugten Lichtbündels, insbesondere eine besonders geringe Minderung der lichtemittierenden Fläche der ersten Lichtquelle oder eine besonders geringe Abschattung des Lichts der ersten Lichtquelle.

Bei einer Vorrichtung, wie sie hier beschrieben ist, kann das Beleuchtungslicht einen Farbwiedergabeindex aufweisen, der um mindestens 10 höher ist als der Farbwiedergabeindex des Lichts der ersten Lichtquelle. Alternativ oder zusätzlich kann der mittlere Farbabstand bei Beleuchtung eines vorbestimmten Satzes von Testfarben mit dem Beleuchtungslicht um mindestens ein Zehntel besser als bei Beleuchtung des vorbestimmten Satzes von Testfarben nur mit Licht der ersten Lichtquelle sein. Alternativ oder zusätzlich kann die Summe der Beträge der Abstände der Weißabgleichsparameter von 1 bei Beleuchtung mit dem Beleuchtungslicht geringer als bei Beleuchtung mit Licht der ersten Lichtquelle sein.

Der Farbwiedergabeindex ist insbesondere der Farbwiedergabeindex Rₐ, der mit den ersten acht Testfarben nach DIN 6169-1:1976-01 (bzw. in CIE 13.3-1995, ISBN 978 3 900 734 57 2) bestimmt wird. Alternativ wird ein problemangepasster Satz von Testfarben zur Ermittlung des Farbwiedergabeindex Rₐ verwendet. Für medizinische Anwendungen umfasst ein solcher problemangepasster Satz von Testfarben insbesondere Gewebefarben. In manchen Fällen kann eine Verbesserung des Werts des Farbwiedergabeindex des Beleuchtungslichts gegenüber dem Licht der ersten Lichtquelle um 15 oder sogar 20 oder eine Verringerung des Farbabstands um ein Sechstel, ein Fünftel oder sogar um ein Viertel erzielt werden.

Auch zur Ermittlung eines mittleren Farbabstands kann ein problemangepasster Satz von Testfarben verwendet werden. Typischerweise wird hierfür eine Testfarbtafel des Unternehmens MacBeth bzw. X-Rite verwendet, wie in CIE 135-1999 (ISBN 3 900 734 97 6 dargelegt. Für medizinische Anwendungen umfasst ein solcher problemangepasster Satz von Testfarben insbesondere Gewebefarben. Die oben erwähnte Verbesserung des Werts des Farbwiedergabeindex des Beleuchtungslichts gegenüber dem Licht der ersten Lichtquelle um 15 oder sogar 20 entspricht näherungsweise einer Verringerung des mittleren Farbabstands bezüglich der genannten Testtafel um ein Sechstel, ein Fünftel oder sogar um ein Viertel.

Die Berechnung des Farbabstands wird standardmäßig innerhalb des CIE LAB Farbraum als euklidischer Abstand zweier Farborte berechnet (Wurzel aus der Summe der Quadrate der Differenzen der Koordinaten). Dies entspricht der CIE Definition des Farbabstands von 1976 (ΔE76). Alternativ hierzu kann die Berechnung auch gemäß neuerer Definitionen erfolgen (beispielsweise ΔE94, CIE 1994 bzw. ΔE00, CIE 2000).

Eine Vorrichtung, wie sie hier beschrieben ist, ist insbesondere dazu ausgebildet, dass das von der zweiten Lichtquelle erzeugte monochromatische Licht innerhalb des Spektrums des von der ersten Lichtquelle erzeugten Lichts liegt.

Das von der zweiten Lichtquelle erzeugte Licht liegt insbesondere insofern innerhalb des breiten Spektrums des von der ersten Lichtquelle erzeugten Lichts, als der oben in Zusammenhang mit der Breite beschriebene symmetrische Wellenlängenbereich des (sehr schmalen) Spektrums der zweiten Lichtquelle vollständig oder überwiegend innerhalb des symmetrischen Wellenlängenbereichs des breiten Spektrums der ersten Lichtquelle liegt. Anders ausgedrückt werden ein erster Wellenlängenbereich und ein zweiter Wellenlängenbereich betrachtet bzw. verglichen. Der erste Wellenlängenbereich ist der zusammenhängende Wellenlängenbereich, innerhalb dessen ein vorbestimmter Anteil (beispielsweise 70 %, 80 % oder 90 %) der gesamten für das menschliche Auge sichtbaren Strahlungsleistung der ersten Lichtquelle liegt, und der symmetrisch im oben genannten Sinne ist. Der zweite Wellenlängenbereich ist der zusammenhängende Wellenlängenbereich, innerhalb dessen der vorbestimmte Anteil der gesamten für das menschliche Auge sichtbaren Strahlungsleistung der zweiten Lichtquelle liegt, und der symmetrisch im oben genannten Sinne ist. Die Bedingung, dass das Spektrum der zweiten Lichtquelle innerhalb des breiten Spektrums der ersten Lichtquelle liegt, ist erfüllt, wenn der zweite Wellenlängenbereich vollständig oder zumindest überwiegend innerhalb des ersten Wellenlängenbereichs liegt.

Eine Vorrichtung. wie sie hier beschrieben ist, kann so ausgebildet sein, dass innerhalb des für das menschliche Auge sichtbaren Spektralbereichs die mittels der Einkopplungseinrichtung in den Strahlengang eingekoppelte Strahlungsleistung höher ist als die Strahlungsleistung des in den Strahlengang einkoppelbaren Lichts der ersten Lichtquelle.

Mittels der hier beschriebenen Vorrichtung kann also neben einer Verbesserung der Farbwiedergabe gleichzeitig die gesamte in den gemeinsamen Strahlengang eingekoppelte Strahlungsleistung verbessert werden. Dies kann insbesondere bei endoskopischen Anwendungen von Vorteil sein, bei denen eine möglichst hohe Strahlungsleistung des Beleuchtungslichts erwünscht ist. Die gerade im Vergleich zu herkömmlichen Gasentladungslampen niedrigere Flächenstrahlungsdichte von Leuchtdioden kann so verbessert werden. Bei einer Vorrichtung, wie sie hier beschrieben ist, kann die zweite Lichtquelle zumindest entweder einen Diodenlaser oder einen anderen Laser umfassen.

Für die Farbwiedergabe vorteilhafte oder optimale Wellenlängen der zweiten Lichtquelle sind insbesondere vom Spektrum des von der ersten Lichtquelle erzeugten Lichts abhängig. Ferner kann die optimale Wellenlänge davon abhängig sein, ob die Farbwiedergabe mit dem ungeschützten, bloßen menschlichen Auge oder mit einem Endoskop, Exoskop oder Mikroskop und/oder mit einer Kamera optimal sein soll. Typische Wellenlängen der zweiten Lichtquelle liegen im Bereich von 630 nm bis 660 nm und von 500 nm bis 530 nm.

Als Faustregel gilt in einigen Fällen, dass die Strahlungsleistung der zweiten Lichtquelle monoton fallend, insbesondere umgekehrt proportional, zur Strahlungsleistung der ersten Lichtquelle bei der von der zweiten Lichtquelle emittierten Wellenlänge ist. Sowohl die Wellenlänge als auch die Strahlungsleistung können empirisch und/oder mittels numerischer Simulation hinsichtlich einer optimalen Farbwiedergabe optimiert werden.

Insbesondere das Licht eines Diodenlasers oder eines anderen Lasers kann aufgrund der Monochromatizität, des Polarisationsgrades und/oder der Gerichtetheit der Emission besonders effizient mit dem Licht der ersten Lichtquelle gemischt werden. Dazu wird beispielsweise eine dichroitisch reflektierende Grenzfläche oder eine polarisationsabhängig reflektierende Grenzfläche oder ein Lichtwellenleiter oder ein kleiner Spiegel, auf den das Licht der zweiten Lichtquelle gebündelt wird, verwendet. Die genannten optischen Elemente können so ausgebildet sein, dass sie Licht der ersten Lichtquelle gar nicht oder lediglich in einem kleinen Wellenlängenbereich oder lediglich in einem kleinen räumlichen Bereich abschatten.

Eine Vorrichtung, wie sie hier beschrieben ist, kann ferner eine dritte Lichtquelle umfassen, wobei die Einkopplungseinrichtung ausgebildet ist, um Licht der ersten Lichtquelle, Licht der zweiten Lichtquelle und Licht der dritten Lichtquelle in den gemeinsamen Strahlengang einzukoppeln, um das Beleuchtungslicht zu erzeugen, und wobei das Spektrum des Beleuchtungslichts eine bessere Farbwiedergabe aufweist als das erste Spektrum und als eine hinsichtlich der Farbwiedergabe optimale Mischung des Lichts der ersten Lichtquelle und des Lichts der zweiten Lichtquelle.

Abhängig von den Eigenschaften der ersten Lichtquelle ist durch Beimischung des Lichts einer dritten Lichtquelle eine weitere, in manchen Fällen deutliche Verbesserung der Farbwiedergabe möglich. Weitere Verbesserungen können mit einer vierten oder weiteren Lichtquellen möglich sein, deren Licht dem Licht der ersten Lichtquelle und dem Licht der zweiten und dritten Lichtquellen beigemischt wird.

Eine Vorrichtung mit drei Lichtquellen kann so ausgebildet sein, dass zumindest entweder das Beleuchtungslicht einen Farbwiedergabeindex aufweist, der um mindestens 15 höher ist als der Farbwiedergabeindex des ersten Spektrums; oder der mittlere Farbabstand bei Beleuchtung eines vorbestimmten Satzes von Testfarben mit dem Beleuchtungslicht um mindestens ein Fünftel besser ist als bei einer Beleuchtung mit Licht der ersten Lichtquelle; oder die Summe der Beträge der Abstände der Weißabgleichsparameter von 1 bei Beleuchtung mit dem Beleuchtungslicht geringer ist, als bei Beleuchtung mit einer hinsichtlich der Farbwiedergabe optimalen Mischung des Lichts der ersten Lichtquelle und des Lichts der zweiten Lichtquelle.

Abhängig von der Qualität des breiten Spektrums der ersten Lichtquelle kann eine Verbesserung des Farbwiedergabeindex um 20 oder sogar 25 und/oder eine Verbesserung des mittleren Farbabstands um ein Fünftel, ein Viertel oder ein Drittel erzielbar sein. Durch das Mischen des Lichts von drei Lichtquellen ist eine Farbwiedergabe erzielbar, die auch hohen oder höchsten Ansprüchen gerecht wird, wie sie insbesondere bei medizinischdiagnostischen Anwendungen vorliegen.

Eine Vorrichtung, wie sie hier beschrieben ist, ist insbesondere ein Untersuchungssystem und umfasst ferner eine Kamera zum Erfassen eines Bilds eines mittels des von der Vorrichtung bereitgestellten Lichts beleuchteten Objekts.

Durch die hier beschriebene Mischung des Lichts zweier oder dreier Lichtquellen kann die Farbwiedergabe nicht nur bei einer unmittelbaren Beobachtung durch das ungeschützte menschliche Auge optimiert werden. Alternativ ist eine Optimierung der Farbwiedergabe bei einer Erfassung mittels der erwähnten Kamera möglich. Die spektralen Empfindlichkeiten der Farbsensoren der Kamera können von denen der Farbrezeptoren des menschlichen Auges deutlich abweichen und tun dies auch bei vielen Kameratypen.

Bei einem Untersuchungssystem, wie es hier beschrieben ist, ist insbesondere die Farbwiedergabe bei Beleuchtung zu untersuchender Objekte mit dem Beleuchtungslicht und Erfassung mittels der Kamera besser als bei Beleuchtung ausschließlich mit dem Licht der ersten Lichtquelle.

Eine Vorrichtung, wie sie hier beschrieben ist, insbesondere ein Untersuchungssystem, kann ferner ein Endoskop oder ein Exoskop oder ein Mikroskop umfassen.

Durch Mischen des Lichts von zwei oder drei Lichtquellen kann die Farbwiedergabe auch unter Berücksichtigung der Wellenlängenabhängigkeit der Transmission eines Endoskops, Exoskops oder Mikroskops verbessert oder optimiert werden, und zwar für eine Beobachtung mit dem bloßen Auge oder für eine Beobachtung mittels einer Kamera.

Ein Verfahren zum Bereitstellen von weißem Beleuchtungslicht für medizinische Anwendungen umfasst folgende Schritte: Erzeugen von Licht mit einem breiten Spektrum mittels einer ersten Lichtquelle, die eine Leuchtdiode umfasst; Erzeugen von monochromatischem Licht mittels einer zweiten Lichtquelle; Einkoppeln von Licht der ersten Lichtquelle und Licht der zweiten Lichtquelle in einen gemeinsamen Strahlengang mittels einer Einkopplungseinrichtung, um Beleuchtungslicht mit einer verbesserten Farbwiedergabe zu erzeugen.

Ein Verfahren, wie es hier beschrieben ist, kann so ausgebildet sein, dass zumindest entweder das Beleuchtungslicht einen Farbwiedergabeindex aufweist, der um mindestens 10 höher ist als der Farbwiedergabeindex des Lichts der ersten Lichtquelle; oder der mittlere Farbabstand bei Beleuchtung eines vorbestimmten Satzes von Testfarben mit dem Beleuchtungslicht um mindestens ein Zehntel besser ist als bei einer Beleuchtung mit Licht der ersten Lichtquelle; oder die Summe der Beträge der Abstände der Weißabgleichsparameter von 1 bei Beleuchtung mit dem Beleuchtungslicht geringer ist als bei Beleuchtung mit Licht der ersten Lichtquelle.

Ein Verfahren, wie es hier beschrieben ist, kann ähnlich variiert werden, wie die hier beschriebenen Vorrichtungen.

### Kurzbeschreibung der Figuren

Nachfolgend werden Ausführungsformen anhand der beigefügten Figuren näher erläutert. Es zeigen:
- Figur 1: eine schematische Darstellung eines Endoskopiesystems;
- Figur 2: eine schematische Darstellung eines weiteren Endoskopiesystems;
- Figur 3: eine schematische Darstellung typischer Wellenlängenabhängigkeiten der Empfindlichkeiten e der Farbkanäle einer Kamera und ein typisches Emissionsspektrum I einer Weißlicht-Leuchtdiode;
- Figur 4: eine schematische Darstellung des mittleren Farbabstands ΔE als Funktion der Wellenlänge λ einer zweiten Lichtquelle;
- Figur 5: eine schematische Darstellung des Farbwiedergabeindex CRI als Funktion der Wellenlänge λ einer zweiten Lichtquelle;
- Figur 6: eine schematische Darstellung eines Weißabgleichparameters B als Funktion der Wellenlänge λ einer zweiten Lichtquelle;
- Figur 7: eine schematische Darstellung eines Weißabgleichparameters G als Funktion der Wellenlänge λ einer zweiten Lichtquelle;
- Figur 8: eine schematische Darstellung eines Weißabgleichparameters R als Funktion der Wellenlänge λ einer zweiten Lichtquelle;
- Figur 9: ein schematisches Flussdiagramm eines Verfahrens zum Bereitstellen von Beleuchtungslicht;
- Figur 10: eine schematische Darstellung einer Lichtquellenvorrichtung;
- Figur 11: eine schematische Darstellung einer weiteren Lichtquellenvorrichtung.

### Beschreibung der Ausführungsformen

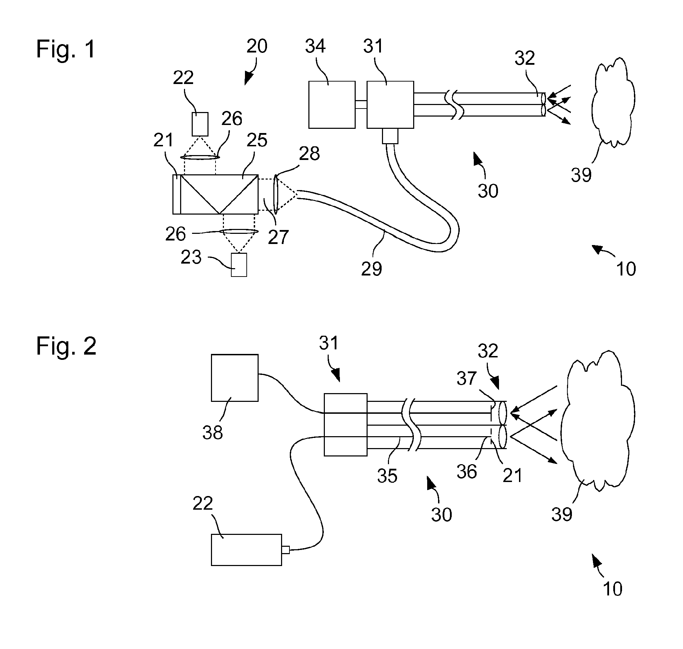

Figur 1 zeigt eine schematische Darstellung eines Endoskopiesystems 10 mit einer Lichtquellenvorrichtung 20. Die Lichtquellenvorrichtung 20 umfasst eine Leuchtdiode 21, einen ersten Laser 22 und einen zweiten Laser 23. Das Licht der Leuchtdiode 21, das Licht des ersten Lasers 22 und das Licht des zweiten Lasers 23 werden über eine Einkopplungseinrichtung 25 in einen Strahlengang 27 eingekoppelt. Bei der dargestellten Lichtquellenvorrichtung 20 umfasst die Einkopplungseinrichtung 25 beispielhaft wellenlängenabhängig oder polarisationsabhängig reflektierende Flächen in einem transparenten Körper, mit dem die Leuchtdiode 21 mechanisch und optisch unmittelbar gekoppelt ist.

Zur Einkopplung des Lichts des ersten Lasers 22 und des Lichts des zweiten Lasers 23 sind beispielhaft jeweils ein Kollimator 26 dargestellt. Die Einkopplungseinrichtung 25 ist ausgebildet, um das Licht der Leuchtdiode 21, das Licht des ersten Lasers 22 und das Licht des zweiten Lasers 23 zu mischen und möglichst vollständig in den Strahlengang 27 einzukoppeln. Die Mischung bzw. Überlagerung des Lichts der Leuchtdiode 21, des Lichts des ersten Lasers 22 und des Lichts des zweiten Lasers 23 wird als Beleuchtungslicht für das Endoskopiesystem 10 in den Beleuchtungs-Strahlengang 27 eingekoppelt. Ein Objektiv 28 bündelt das Beleuchtungslicht im Beleuchtungs-Strahlengang 27 auf eine Lichteintrittsfläche eines Lichtleitkabels 29, das ebenfalls Bestandteil des Beleuchtungs-Strahlengangs ist.

Das Lichtleitkabel 29 koppelt die Lichtquellenvorrichtung 20 mit einem Endoskop 30, insbesondere mit einem proximalen Ende 31 des Endoskops 30. Hier nicht näher beschriebene optische Einrichtungen sind zur Übertragung des Beleuchtungslichts vom proximalen Ende 31 des Endoskops zu dessen distalem Ende 32 ausgebildet. Eine Kamera 34 ist mit dem proximalen Ende 31 des Endoskops 30 optisch und mechanisch gekoppelt.

Von der Lichtquellenvorrichtung 20 erzeugtes Beleuchtungslicht wird mittels des Lichtleitkabels 29 und der erwähnten optischen Einrichtungen des Endoskops 30 zum distalen Ende desselben übertragen und tritt dort aus, um ein Objekt 39 zu beleuchten. Das Objekt 39 ist beispielsweise Gewebe oder ein anderes medizinisches Objekt, das mittels des Endoskopiesystems 10 untersucht wird. Von dem Objekt 39 remittiertes Beleuchtungslicht, das auf das distale Ende 32 des Endoskops 30 fällt, wird zum proximalen Ende 31 des Endoskops 30 und zur Kamera 34 übertragen, um dort ein Bild des Objekts 39 zu erzeugen.

Figur 2 zeigt eine schematische Darstellung eines weiteren Endoskopiesystems 10, das in einigen Merkmalen dem oben anhand der Figur 1 dargestellten Endoskopiesystem ähnelt. Abweichend von dem oben anhand der Figur 1 dargestellten Endoskopiesystem ist die Lichtquellenvorrichtung teilweise in das Endoskop 30 integriert. Die Leuchtdiode 21 ist am distalen Ende 32 des Endoskops 30 angeordnet. Der erste Laser 22 ist über einen Lichtwellenleiter 35 mit dem distalen Ende 32 des Endoskops 30 gekoppelt. Das distale Ende 36 des Lichtwellenleiters 35 ist unmittelbar neben der Leuchtdiode 21 oder in einer Öffnung in der Leuchtdiode 21 angeordnet. Der Lichtwellenleiter 35 bzw. dessen distales Ende 36, an dem vom Laser 22 erzeugtes Licht unmittelbar neben der Leuchtdiode 21 oder in einer Öffnung in der Leuchtdiode 21 austritt, bildet eine Einkopplungseinrichtung zum Mischen des Lichts der Leuchtdiode 21 und des Lichts des Lasers 22.

Abweichend von dem oben anhand der Figur 1 dargestellten Endoskopiesystem ist anstelle einer mit dem proximalen Ende 31 des Endoskops 30 gekoppelten separaten Kamera 34 ein lichtempfindlicher Sensor 37 am distalen Ende des Endoskops 10 angeordnet und über Leitungen mit einer Kamerasteuerung 38 gekoppelt.

Abweichend von der Darstellung in Figur 2 kann die Leuchtdiode 21 am proximalen Ende 31 des Endoskops 30 angeordnet sein. In diesem Fall ist beispielsweise ein Lichtwellenleiter oder ein ungeordnetes oder geordnetes Bündel von Lichtwellenleitern zur Übertragung von Beleuchtungslicht vom proximalen Ende 31 zum distalen Ende 32 des Endoskops 30 vorgesehen.

Abweichend von der Darstellung in Figur 2 kann der lichtempfindliche Sensor 37 am proximalen Ende 31 des Endoskops 30 angeordnet sein. In diesem Fall ist beispielsweise eine Stablinsenoptik oder ein geordnetes Bündel von Lichtwellenleitern zur Übertragung von vom Objekt 39 remittiertem Beleuchtungslicht vom distalen Ende 32 zum proximalen Ende 31 des Endoskops 30 vorgesehen.

Abweichend von der Darstellung in Figur 2 können ferner der Laser 22 und/oder die Kamerasteuerung 38 in das Endoskop 30 integriert sein, insbesondere an dessen proximalem Ende 31.

Ähnlich wie oben anhand der Figuren 1 und 2 für ein Endoskopiesystem dargestellt, kann eine Lichtquellenvorrichtung alternativ zur Bereitstellung von Beleuchtungslicht für exoskopische, mikroskopische oder andere medizinische, insbesondere medizinischdiagnostische, Anwendungen ausgebildet sein. Dazu kann die Lichtquellenvorrichtung für eine Kombination mit einem Exoskop oder einem Mikroskop ausgebildet oder mit einem Exoskop oder einem Mikroskop teilweise oder vollständig integriert sein.

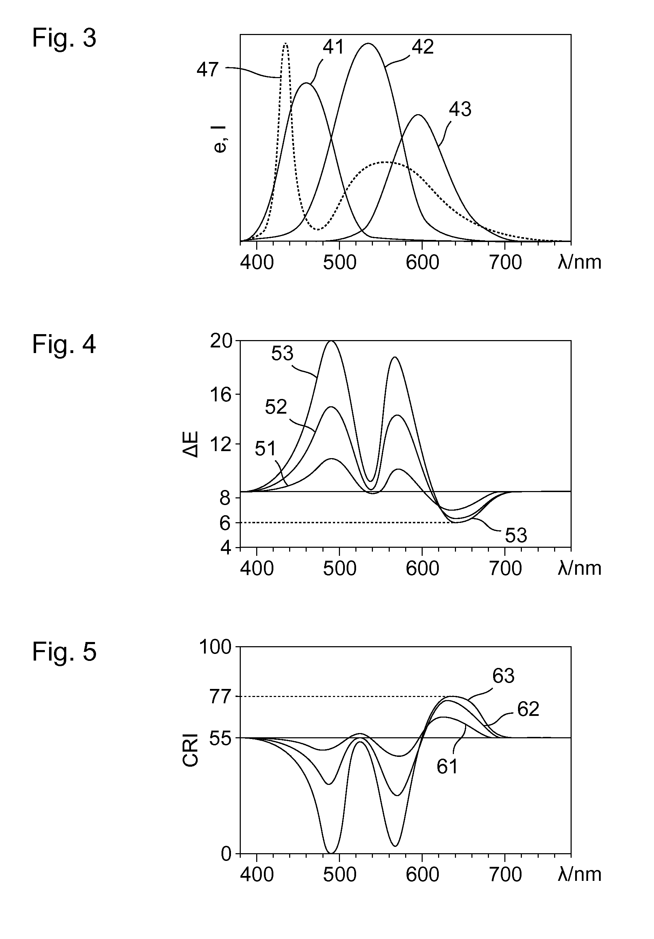

Figur 3 zeigt eine schematische Darstellung der Wellenlängenabhängigkeiten der Empfindlichkeiten e der Farbkanäle einer typischen Kamera und des Emissionsspektrums einer typischen Weißlicht-Leuchtdiode. Der Abszisse ist die Wellenlänge λ zugeordnet, der Ordinate die Empfindlichkeit e bzw. die Intensität I in willkürlichen Einheiten. Die Empfindlichkeit 41 des blauen Farbkanals weist ein Maximum zwischen 440 nm und 480 nm auf. Die Empfindlichkeit 42 des grünen Farbkanals weist ein Maximum zwischen 500 nm und 560 nm auf. Die Empfindlichkeit des roten Farbkanals weist ein Maximum zwischen 580 nm und 640 nm auf.

Das Emissionsspektrum 47 der Weißlicht-Leuchtdiode weist ein Maximum bei 430 nm auf, das unmittelbar von einem blaues Licht emittierenden Halbleiterübergang erzeugt wird. Ferner weist das Emissionsspektrum der Weißlicht-Leuchtdiode ein weiteres, breites Maximum zwischen ca. 500 nm und ca. 600 nm auf, das von der Fluoreszenz oder Phosphoreszenz eines Farbstoffs erzeugt wird, der durch den bei 430 nm emittierenden Halbleiterübergang angeregt wird. Es ist erkennbar, dass das Emissionsspektrum 47 der Weißlicht-Leuchtdiode 21 mit den beiden ausgeprägten Maxima und dem ebenso ausgeprägten Minimum dazwischen von einem Planck-Spektrum deutlich abweicht.

Figur 4 zeigt eine schematische Darstellung der Abhängigkeit eines mittleren Farbabstands ΔE bei Beleuchtung eines vorbestimmten Satzes von Testfarben durch eine Mischung des Lichts der Weißlicht-Leuchtdiode 21 mit dem oben anhand der Figur 3 dargestellten Emissionsspektrum 47 und des Lasers 22 als Funktion der Wellenlänge λ und der Intensität des Laserlichts. Der Abszisse ist die Wellenlänge λ des vom Laser emittierten Lichts zugeordnet, der Ordinate ist der mittlere Farbabstand ΔE zugeordnet. Dargestellt sind der mittlere Farbabstand 51 bei einer ersten, geringen Intensität des Lichts des Lasers 22, der mittlere Farbabstand 52 bei einer zweiten, mittleren Intensität des Lichts des Lasers 22 und der mittlere Farbabstand 53 bei einer dritten, optimalen Intensität des Lichts des Lasers 23. Tatsächlich kann der mittlere Farbabstand für beliebige Intensitäten und beliebige Wellenlängen λ des Lichts des Lasers 22 berechnet bzw. simuliert werden.

Der mittlere Farbabstand ΔE beträgt für die Weißlicht-Leuchtdiode 21 mit dem oben anhand der Figur 3 dargestellten Emissionsspektrums 47 allein ohne Beimischung des Lichts eines Lasers ca. 8,5. Bei Bemischung des Lichts des Lasers 22 bei der dritten, optimalen Intensität und einer Emissionswellenlänge des Lasers 22 von ca. 640 nm beträgt der mittlere Farbabstand ΔE nur noch ca. 6.

Figur 5 zeigt eine schematische Darstellung des Farbwiedergabeindex CRI (Color Rendering Index) für die ersten acht Testfarben nach DIN 6169-1:1976-01 für Beleuchtungslicht, das durch eine Mischung des Lichts der Weißlicht-Leuchtdioden 21 mit dem oben anhand der Figur 3 dargestellten Emissionsspektrum 47 und dem Licht des Lasers 22 erzeugt wird, als Funktion der Wellenlänge λ und der Intensität des vom Laser 22 erzeugten Lichts. Der Abszisse ist die Wellenlänge λ des vom Laser 22 erzeugten Lichts zugeordnet, der Ordinate der Farbwiedergabeindex CRI. Dargestellt ist der Farbwiedergabeindex 61 bei der ersten, geringen Intensität des Lichts des Lasers 22, der Farbwiedergabeindex 62 bei der zweiten, mittleren Intensität des Lichts des Lasers 22 und der Farbwiedergabeindex 63 bei der dritten, optimalen Intensität des Lichts des Lasers 22. Der Farbwiedergabeindex CRI des von der Weißlicht-Leuchtdiode 21 erzeugten Lichts mit dem oben anhand der Figur 3 dargestellten Spektrums 47 beträgt ca. 56. Bei Beimischung des Lichts eines bei 640 nm emittierenden Lasers mit optimaler Intensität beträgt der Farbwiedergabeindex CRI ca. 77.

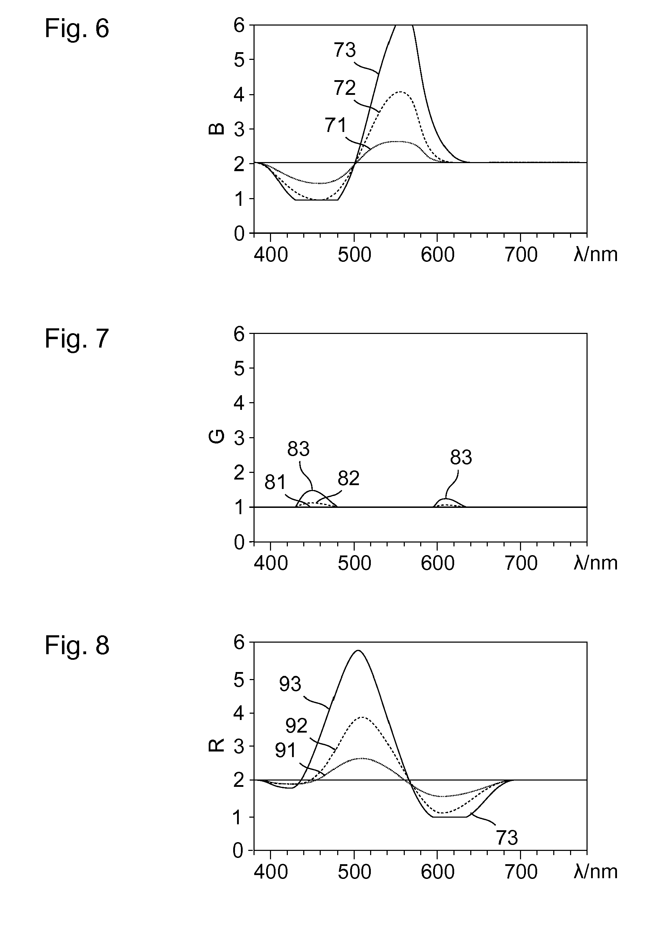

Die Figuren 6, 7 und 8 zeigen schematische Darstellungen der Weißabgleichparameter B, G, R für den blauen, grünen bzw. roten Farbkanal bei Beleuchtung einer unbunten Testfarbe mit Beleuchtungslicht, das durch Mischung des von der Weißlicht-Leuchtdiode 21 erzeugten Lichts mit dem oben anhand der Figur 3 dargestellten Spektrum 47 und des Lichts des Lasers 22 erzeugt wird, in Abhängigkeit von der Wellenlänge λ und der Intensität der Emission des Lasers 22. Der Abszisse ist jeweils die Wellenlänge λ zugeordnet, der Ordinate der Weißabgleichparameter B, G bzw. R.

Figur 6 zeigt den Weißabgleichparameter 71 für den blauen Farbkanal bei der ersten, geringen Intensität des vom Laser 22 erzeugten Lichts, den Weißabgleichparameter 72 für den blauen Farbkanal bei der zweiten, mittleren Intensität des vom Laser 22 erzeugten Lichts und den Weißabgleichparameter 73 für den blauen Farbkanal bei der dritten, optimalen Intensität des vom Laser 22 erzeugten Lichts.

Figur 7 zeigt den Weißabgleichparameter 81 für den grünen Farbkanal bei der ersten, geringen Intensität des vom Laser 22 erzeugten Lichts, den Weißabgleichparameter 82 für den grünen Farbkanal bei der zweiten, mittleren Intensität des vom Laser 22 erzeugten Lichts und den Weißabgleichparameter 83 für den grünen Farbkanal bei der dritten, optimalen Intensität des vom Laser 22 erzeugten Lichts.

Figur 8 zeigt den Weißabgleichparameter 91 für den roten Farbkanal bei der ersten, geringen Intensität des vom Laser 22 erzeugten Lichts, den Weißabgleichparameter 92 für den roten Farbkanal bei der zweiten, mittleren Intensität des vom Laser 22 erzeugten Lichts und den Weißabgleichparameter 93 für den roten Farbkanal bei der dritten, optimalen Intensität des vom Laser 22 erzeugten Lichts.

Bei dem in den Figuren 6 bis 8 dargestellten Beispiel kann jeder der drei Weißabgleichparameter B, G, R definitionsgemäß nicht kleiner als 1 sein. Ein Optimum, insbesondere hinsichtlich der erzielbaren Dynamik, liegt vor, wenn alle Weißabgleichparameter B = G = R = 1,0 betragen. Dies setzt Beleuchtung mit einem Beleuchtungslicht mit einem hinsichtlich Farbtemperatur und Farbwiedergabe optimalen Spektrum voraus. Von diesem Optimum ist das von der Weißlicht-Leuchtdiode 21 erzeugte Licht mit dem oben anhand der Figur 3 dargestellten Spektrum 47 erkennbar weit entfernt. Für eine Beleuchtung der unbunten Testfarbe mit dem von der Weißlicht-Leuchtdiode 21 erzeugten Licht mit dem oben anhand der Figur 3 dargestellten Spektrum 47 betragen der Weißabgleichparameter B für den blauen Farbkanal ca. 2,1, der Weißabgleichparameter G für den grünen Farbkanal 1,0 und der Weißabgleichparameter R für den roten Farbkanal ca. 2,0.

Bei Beleuchtungslicht, das bei einem optimalen Mischungsverhältnis des Lichts der Weißlicht-Leuchtdiode 21 mit dem oben anhand der Figur 3 dargestellten Spektrum 47 und Laserlicht mit ca. 640 nm erzeugt wird, beträgt der Weißabgleichparameter B für den blauen Farbkanal weiterhin ca. 2,1, der Weißabgleichparameter G für den grünen und der Weißabgleichparameter R für den roten Farbkanal betragen jeweils ca. 1,0. Bei 640 nm und der optimalen Intensität ist somit auch die Summe der Beträge der Abstände der Weißabgleichparameter B, G, R vom Idealwert 1 geringer als für das von der Weißlicht-Leuchtdiode 21 allein erzeugte Licht mit dem oben anhand der Figur 3 dargestellten Spektrum 47.

Eine weitere Verbesserung der Farbwiedergabe, die sich in einem weiter verringerten mittleren Farbabstand ΔE, einem weiter erhöhten Farbwiedergabeindex und noch besser an den Idealwert 1 angenäherten Weißabgleichparametern B, G, R ausdrückt, ist durch Zumischen des Lichts eines zweiten Lasers 23 möglich. Anhand der Figuren 4 und 5 ist erkennbar, dass die optimale Wellenlänge λ des zweiten Lasers im Bereich von 530 nm bis 540 nm liegt. Tatsächlich können bei dem dargestellten Beispiel durch zusätzliches Zumischen des Lichts des zweiten Lasers 23 mit einer Wellenlänge λ von 540 nm der mittlere Farbabstand auf ca. 4,5 verringert und der Farbwiedergabeindex auf ca. 82 erhöht werden.

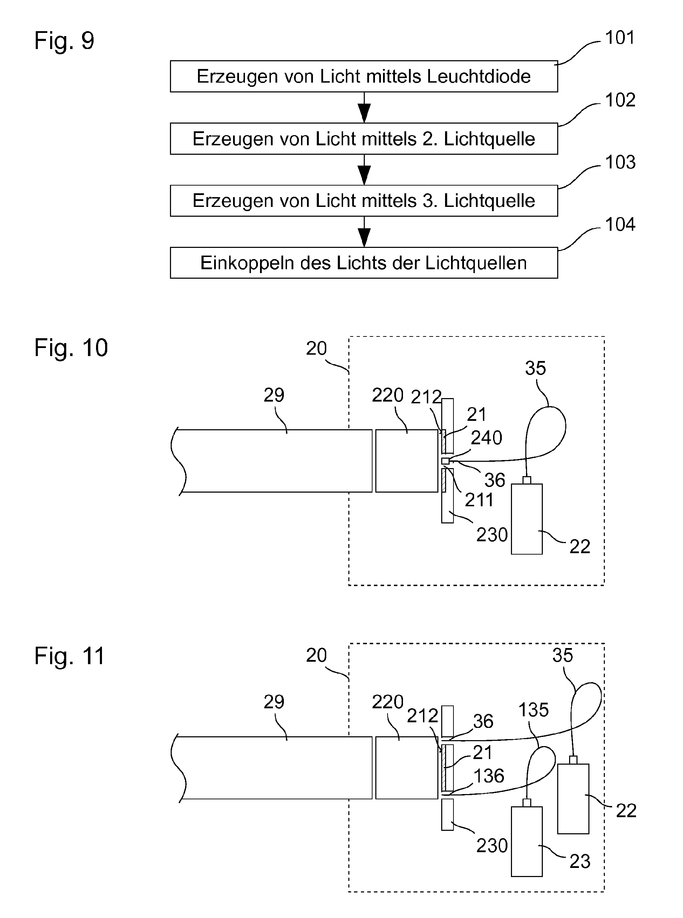

Figur 9 zeigt ein schematisches Flussdiagramm eines Verfahrens zum Bereitstellen von weißem Beleuchtungslicht. Obwohl dieses Verfahren auch mittels einer Lichtquellenvorrichtung durchführbar ist, die sich von der oben anhand der Figuren 1 bis 3 dargestellten Lichtquellenvorrichtung unterscheidet, werden nachfolgend Bezugszeichen aus den Figuren 1 bis 8 verwendet, um das Verständnis zu fördern.

Bei einem ersten Schritt 101 wird mittels einer ersten Lichtquelle, die eine Leuchtdiode 21 umfasst, Licht mit einem ersten Spektrum 47 erzeugt. Bei einem zweiten Schritt 102 wird mittels einer zweiten Lichtquelle Licht mit einem zweiten Spektrum erzeugt, wobei die Halbwertsbreite des zweiten Spektrums höchstens halb so groß ist wie die Halbwertsbreite des ersten Spektrums 47. Bei einem optionalen dritten Schritt 103 wird mittels einer dritten Lichtquelle 23 Licht mit einem dritten Spektrum erzeugt, wobei die Halbwertsbreite des dritten Spektrums nicht größer als die Halbwertsbreite des zweiten Spektrums ist. Die zweite Lichtquelle 22 und ggf. die dritte Lichtquelle 23 umfassen jeweils insbesondere einen Diodenlaser oder einen anderen Laser. Bei einem vierten Schritt 104 werden mittels einer Einkopplungseinrichtung 25, 35 Licht der ersten Lichtquelle 21, Licht der zweiten Lichtquelle 22 und ggf. Licht der dritten Lichtquelle 23 gemischt, um Beleuchtungslicht mit einer verbesserten Farbwiedergabe zu erzeugen.

Nachfolgend werden anhand der Figuren 10 und 11 zwei weitere Lichquellenvorrichtungen beschrieben, mittels derer weißes Licht für medizinische oder boroskopische Anwendungen bereitgestellt werden kann. Die anhand der Figuren 10 und 11 dargestellten Lichtquellevorrichtungen können beispielsweise bei dem oben anhand der Figur 1 dargestellten Endoskopiesystem die dort gezeigte Lichtquellenvorrichtung ersetzen.

Figur 10 zeigt eine Lichtquellenvorrichtung 20 mit einer Leuchtdiode oder einem Array von Leuchtdioden 21. Nachfolgend wird zunächst davon ausgegangen, dass die Lichtquellenvorrichtung 20 eine einzelne Leuchtdiode 21 aufweist.

Die Leuchtdiode 21 weist eine lichtemittierende Fläche 212 auf, die eben oder im Wesentlichen eben ist. Die Leuchtdiode 21 kann eine anorganische oder organische Leuchtdiode sein. Die Leuchtdiode 21 kann einen lichtemittierenden Halbleiterübergang, der zur Emission von blauem oder violettem Licht ausgebildet ist, und eine phosphoreszierende oder fluoreszierende Schicht an der lichtemittierenden Oberfläche 212 aufweisen. Die phosphoreszierende oder fluoreszierende Schicht an der lichtemittierenden Fläche 212 kann ausgebildet sein, um einen Teil des blauen oder violetten Lichts zu absorbieren und durch Fluoreszenz oder Phosphoreszenz Licht im grünen, gelben und/oder roten Spektralbereich zu emittieren. Mit diesem Aufbau ist die Leuchtdiode 21 ausgebildet, um vom menschlichen Auge als weiß empfundenes Licht zu emittieren.

Alternativ können mehrere Halbleiterübergänge vorgesehen sein, die zur Emission von unterschiedlichen Spektren ausgebildet sind, wobei die Spektren und die Strahlungsleistungen der einzelnen Halbleiterübergänge so ausgebildet sind, dass insgesamt ein vom menschlichen Auge als weiß wahrgenommenes Licht emittiert wird. Diese Halbleiterübergänge können in einem Array angeordnet sein.

Der lichtemittierenden Fläche 212 der Leuchtdiode 21 gegenüber ist ein Lichtleitkörper 220 angeordnet. Der Lichtleitkörper kann, wie in Figur 10 angedeutet, von der lichtemittierenden Oberfläche 212 der Leuchtdiode 21 beabstandet sein. Alternativ kann der Lichtleitkörper 220 flächig an der lichtemittierenden Fläche 212 der Leuchtdiode 21 anliegen oder mit dieser verkittet sein. Die der lichtemittierenden Fläche 212 der Leuchtdiode 21 gegenüberliegende Fläche des Lichtleitkörpers 220 ist Lichteintrittsfläche, die von der lichtemittierenden Fläche 212 der Leuchtdiode 21 abgewandte Fläche des Lichtleitkörpers 220 ist Lichtaustrittsfläche. Die Lichteintrittsfläche und die Lichtaustrittsfläche des Lichtleitkörpers 220 können durch eine Beschichtung entspiegelt sein, insbesondere wenn sie von der lichtemittierenden Oberfläche 212 der Leuchtdiode bzw. von anderen optischen Elementen beabstandet sind. Weitere Oberflächen des Lichtleitkörpers 220 können verspiegelt sein.

Die Leuchtdiode 21 weist eine Öffnung 211 in Form eines Durchgangslochs, das sich von der lichtemittierenden Fläche 212 bis zu einer gegenüberliegenden Rückseite der Leuchtdiode 21 erstreckt, auf. Die Leuchtdiode 21 ist an einem Kühlkörper 230 angeordnet, der eine mit der Öffnung 211 der Leuchtdiode 21 korrespondierende Öffnung aufweist. Aufgrund der Öffnung 211 ist die lichtemittierende Fläche 212 der Leuchtdiode 21 nicht einfach, sondern mehrfach zusammenhängend bzw. weist ein entsprechendes Loch auf.

Die Lichtquellenvorrichtung 20 umfasst ferner einen Laser 22 und einen Lichtwellenleiter 35. Der Lichtwellenleiter 35 koppelt den Laser 22 mit einer Linse 240, die in oder an der Öffnung 211 in der Leuchtdiode 71 angeordnet ist. Die Linse 240 ist beispielsweise eine Gradientenindexlinse. Die von dem Lichtwellenleiter 35 abgewandte Oberfläche der Linse 240 ist parallel zur lichtemittierenden Oberfläche 212 der Leuchtdiode 21 angeordnet und liegt insbesondere in einer Ebene oder im Wesentlichen in einer Ebene mit der lichtemittierenden Fläche 212 der Leuchtdiode 21. Die Linse 240 ermöglicht eine Formung des vom Laser 22 erzeugten und vom Lichtwellenleiter 35 ausgehenden Lichtbündels, insbesondere eine Beeinflussung von dessen Divergenz.

Gegenüber der Lichtaustrittsfläche des Lichtleitkörpers 220 kann ein Ende des bereits oben anhand der Figur 1 beschriebenen Lichtleitkabels 29 angeordnet werden. Der Lichtleitkörper 220 oder der Lichtleitkörper 220 und das Lichtleitkabel 29 gemeinsam bilden einen gemeinsamen Strahlengang, in den sowohl von der Leuchtdiode 21 ausgehendes Licht als auch vom Laser 22 ausgehendes Licht eingekoppelt wird.

Die Ausdehnung des von der Leuchtdiode 21 erzeugten Lichtbündels und seine Querschnittsfläche sind im Wesentlichen durch die lichtemittierende Fläche 212 der Leuchtdiode 21 und die Querschnitte des Lichtleitkörpers 220 und ggf. des Lichtleitkabels 29 bestimmt. Die Hauptausbreitungsrichtung des von der Leuchtdiode 21 erzeugten Lichtbündels im Beleuchtungsstrahlengang entspricht im Wesentlichen den Flächennormalen der lichtemittierenden Fläche 212 der Leuchtdiode 21, der Lichteintrittsfläche und der Lichtaustrittsfläche des Lichtleitkörpers 220 und der Längsachse bzw. Längsrichtung des Lichtleitkabels 29.

Vom Laser 22 erzeugtes Licht wird in den Lichtwellenleiter 35 eingekoppelt, von diesem zu der Linse 240 übertragen und von der Linse 240 in den Lichtleitkörper 220 und damit in den Beleuchtungsstrahlengang eingekoppelt. Der Ort der Einkopplung des vom Laser 22 erzeugten Licht in den gemeinsamen Strahlengang ist die Öffnung 211 in der Leuchtdiode 21, insbesondere derjenige Abschnitt der durch die lichtemittierende Fläche 212 der Leuchtdiode 21 definierten Ebene, der innerhalb der Öffnung 211 liegt. Im weiteren Sinne können auch die Lichtaustrittsfläche der Linse 240 oder der der Öffnung 211 in der Leuchtdiode 21 gegenüberliegende Abschnitt der Lichteintrittsfläche des Lichtleitkörpers 220 als Ort der Einkopplung angesehen werden.

Am Ort der Einkopplung weist das vom Laser 22 erzeugte und vom Lichtwellenleiter 35 übertragene Lichtbündel eine Querschnittsfläche auf, die höchstens der Querschnittsfläche der Öffnung 211 in der Leuchtdiode 21 entspricht. Am gleichen Ort bzw. in der gleichen Ebene entspricht die Querschnittsfläche des von der Leuchtdiode 21 ausgehenden Lichtbündels im Wesentlichen der lichtemittierenden Fläche 212 der Leuchtdiode 21. Damit ist die Querschnittsfläche des vom Laser 22 erzeugten Lichtbündels wesentlich kleiner als die Querschnittsfläche des von der Leuchtdiode 21 erzeugten Lichtbündels. Ab dem Ort der Einkopplung lichtstromabwärts sind die Hauptrichtungen des von der Leuchtdiode 21 ausgehenden Lichtbündels und des von der Laserdiode erzeugten Lichtbündels gleich oder im Wesentlichen gleich. Die Lichtbündel können sich jedoch in ihrer Divergenz deutlich unterscheiden.

Der Laser 22 ist ausgebildet, um grünes, gelbes oder rotes Licht zu emittieren, welches das von der Leuchtdiode 21 emittierte Spektrum ergänzt. Dazu werden die Leuchtdiode 21 und der Laser 22 gleichzeitig betrieben. Mit einem gleichzeitigen Betrieb der Leuchtdiode 21 und des Lasers 22 ist auch ein Betrieb gemeint, bei dem die Leuchtdiode 22 und der Laser 22 nur teilweise gleichzeitig oder so schnell alternierend Licht emittieren, dass die unterschiedlichen Beleuchtungszustände vom menschlichen Auge bzw. von der Kamera 34 nicht mehr zeitlich aufgelöst werden können. Beispielsweise werden die Leuchtdiode 21 und der Laser 22 so schnell alternierend betrieben, dass innerhalb des Belichtungsintervalls eines einzigen von der Kamera 34 erfassten Bilds oder Halbbilds das Objekt 39 einmal oder mehrmals mit Licht der Leuchtdiode 21 und einmal oder mehrmals mit Licht des Laser 22 beleuchtet wird.

Durch die Ergänzung des von der Leuchtdiode 21 emittierten Lichts durch das vom Laser 22 emittierte Licht kann das Spektrum des das Objekt 39 beleuchtenden Beleuchtungslichts eine verbesserte Farbwiedergabe aufweisen bzw. ermöglichen

Wie bereits erwähnt, kann die Lichtquellenvorrichtung 20 anstelle einer einzelnen Leuchtdiode 21 auch ein Array von Leuchtdioden mit einer ein- oder zweidimensionalen regelmäßigen oder unregelmäßigen Anordnung von Leuchtdioden umfassen. In diesem Fall kann die Linse 240 in einem Zwischenraum zwischen zwei Leuchtdioden angeordnet sein, wobei das Bezugszeichen 240 anstelle einer Öffnung in einer Leuchtdiode einen Zwischenraum zwischen zwei Leuchtdioden bezeichnet.

Figur 11 zeigt eine schematische Darstellung einer Lichtquellenvorrichtung 20, die in mehreren Merkmalen der oben anhand der Figur 10 dargestellten Lichtquellenvorrichtungen ähnlich ist. Im Unterschied zu dieser sind zwei Laser 22, 23 mit zwei Lichtwellenleitern 35, 135 vorgesehen. Anders als bei der oben anhand der Figur 10 dargestellten Lichtquellenvorrichtung sind die distalen Enden 36, 136 der Lichtwellenleiter 35, 135 jedoch nicht in einer Öffnung, sondern am Rand der Leuchtdiode 21 angeordnet. Insbesondere sind die distalen Enden 36, 136 der Lichtwellenleiter 35, 135 an gegenüberliegenden Abschnitten des Rands der Leuchtdiode 21 angeordnet. Diese gegenüberliegenden Abschnitte können gerade oder gekrümmt sein, insbesondere konkav.

Vorteile dieser Anordnung und weitere Merkmale können denen der oben anhand der Figuren 1, 2 und 10 beschriebenen Lichtquellenvorrichtungen entsprechen. Im Unterschied zu der oben anhand der Figur 10 dargestellten Lichtquellenvorrichtung sind keine Linsen an den distalen Enden 36, 136 der Lichtwellenleiter 35, 135 vorgesehen. Abweichend von der Darstellung in Figur 11 können jedoch ähnlich wie bei der oben anhand der Figur 10 dargestellten Lichtquellenvorrichtung und deren Varianten Linsen an den distalen Enden 36, 136 der Lichtwellenleiter 35, 135 vorgesehen sein.

Bei den oben anhand der Figuren 10 und 11 dargestellten Lichtquellenvorrichtungen kann das Licht eines Lasers 22, 23 mittels mehrerer Lichtwellenleiter, deren distalen Enden in mehreren verschiedenen Öffnungen 211 oder an verschiedenen Stellen am Rand der Leuchtdiode 21 angeordnet sind, in den gemeinsamen Strahlengang eingekoppelt werden. Anstelle von zwei Lasern 22, 23, deren Licht mittels zweier Lichtwellenleiter 35, 135 in den Beleuchtungsstrahlengang eingekoppelt wird, kann das Licht von drei oder mehr Lasern über eine entsprechende Anzahl von Lichtwellenleitern in den gemeinsamen Strahlengang eingekoppelt werden.

Die oben anhand der Figuren 10, 11 dargestellten Lichtquellenvorrichtungen weisen jeweils einen Lichtleitkörper 220 auf. Abweichend davon und alternativ dazu können die Lichtquellenvorrichtungen 20 jeweils ohne einen Lichtleitkörper 220 ausgebildet sein. In diesem Fall kann das Lichtleitkabel 29 lösbar oder permanent (beispielsweise mittels eines optisch transparenten Kitts) mit der lichtemittierenden Fläche 212 der Leuchtdiode 21 und den distalen Enden 36, 136 der Lichtwellenleiter 35, 135 bzw. daran angeordneten Linsen optisch gekoppelt oder optisch gekoppelt und mechanisch verbunden sein.

Ein Lichtleitkörper 220 kann zur Homogenisierung bzw. Mischung der von der Leuchtdiode 21 und dem oder den Lasern 22, 23 erzeugten Lichtbündel beitragen. Dies gilt bei einer Anordnung des oder der distalen Enden 36, 136 von Lichtwellenleitern 35, 135 in Öffnungen 211 und insbesondere bei einer Anordnung des oder der distalen Enden 36, 136 am Rand der Leuchtdiode 21. Dazu weist der Lichtleitkörper 220 beispielsweise leicht opake Eigenschaften auf.

Bei den oben dargestellten Lichtquellenvorrichtungen und Verfahren können die Leuchtdiode 21 und die Laser 22, 23 gleichzeitig oder abwechselnd betrieben werden. Insbesondere können die Leuchtdiode 21 und die Laser 22, 23 nur teilweise gleichzeitig oder so schnell alternierend Licht emittieren, dass die unterschiedlichen Beleuchtungszustände vom menschlichen Auge bzw. von der Kamera 34 nicht mehr zeitlich aufgelöst werden können. Beispielsweise werden die Leuchtdiode 21 und die Laser 22, 23 so schnell alternierend betrieben, dass innerhalb des Belichtungsintervalls eines einzigen von der Kamera 34 erfassten Bilds oder Halbbilds das Objekt 39 einmal oder mehrmals mit Licht der Leuchtdiode 21 und einmal oder mehrmals mit Licht der Laser 22, 23 beleuchtet wird.

### Bezugszeichen

- 10: Endoskopiesystem
- 20: Lichtquellenvorrichtung
- 21: Leuchtdiode
- 22: erster Laser
- 23: zweiter Laser
- 25: Einkopplungseinrichtung
- 26: Kollimator
- 27: Strahlengang
- 28: Objektiv
- 29: Lichtleitkabel zur Kopplung der Lichtquellenvorrichtung 20 und des Endoskops 30
- 30: Endoskop
- 31: proximales Ende des Endoskops 20
- 32: distales Ende des Endoskops 20
- 34: Kamera am Endoskop 30
- 35: Lichtwellenleiter
- 36: distales Ende des Lichtwellenleiter 35
- 37: lichtempfindlicher Sensor
- 38: Kamerasteuerung
- 39: Objekt
- 41: spektrale Empfindlichkeit e des blauen Farbkanals der Kamera 34
- 42: spektrale Empfindlichkeit e des grünen Farbkanals der Kamera 34
- 43: spektrale Empfindlichkeit e des roten Farbkanals der Kamera 34
- 47: Emissionsspektrum der Leuchtdiode
- 51: mittlerer Farbabstand bei geringer Intensität des vom Laser 22 erzeugten Lichts
- 52: mittlerer Farbabstand bei mittlerer Intensität des vom Laser 22 erzeugten Lichts
- 53: mittlerer Farbabstand bei optimaler Intensität des vom Laser 22 erzeugten Lichts
- 61: Farbwiedergabeindex bei geringer Intensität des vom Laser 22 erzeugten Lichts
- 62: Farbwiedergabeindex bei mittlerer Intensität des vom Laser 22 erzeugten Lichts
- 63: Farbwiedergabeindex bei optimaler Intensität des vom Laser 22 erzeugten Lichts
- 71: Weißabgleichparameter B für den blauen Farbkanal bei geringer Intensität des vom Laser 22 erzeugten Lichts
- 72: Weißabgleichparameter B für den blauen Farbkanal bei mittlerer Intensität des vom Laser 22 erzeugten Lichts
- 73: Weißabgleichparameter B für den blauen Farbkanal bei optimaler Intensität des vom Laser 22 erzeugten Lichts
- 81: Weißabgleichparameter G für den grünen Farbkanal bei geringer Intensität des vom Laser 22 erzeugten Lichts
- 82: Weißabgleichparameter G für den grünen Farbkanal bei mittlerer Intensität des vom Laser 22 erzeugten Lichts
- 83: Weißabgleichparameter G für den grünen Farbkanal bei optimaler Intensität des vom Laser 22 erzeugten Lichts
- 91: Weißabgleichparameter R für den roten Farbkanal bei geringer Intensität des vom Laser 22 erzeugten Lichts
- 92: Weißabgleichparameter R für den roten Farbkanal bei mittlerer Intensität des vom Laser 22 erzeugten Lichts
- 93: Weißabgleichparameter R für den roten Farbkanal bei optimaler Intensität des vom Laser 22 erzeugten Lichts
- 101: erster Schritt
- 102: zweiter Schritt
- 103: dritter Schritt
- 104: vierter Schritt
- 135: Lichtwellenleiter
- 136: distales Ende des Lichtwellenleiter 135
- 211: Öffnung in der Leuchtdiode 21
- 212: lichtemittierende Fläche der Leuchtdiode 21
- 220: Lichtleitkörper
- 230: Kühlkörper
- 240: Linse

## Patentansprüche

1. Vorrichtung (10; 20) zur Bereitstellung von weißem Beleuchtungslicht für medizinische und boroskopische Anwendungen, mit:
einer ersten Lichtquelle (21) mit einer Leuchtdiode, zur Emission von Licht mit einem breiten Spektrum (47);
einer zweiten Lichtquelle (22) zur Emission von monochromatischem Licht;
einer Einkopplungseinrichtung (25; 35, 135) zum Einkoppeln von Licht der ersten Lichtquelle (21) und Licht der zweiten Lichtquelle (22) in einen gemeinsamen Strahlengang (27), um Beleuchtungslicht mit einer verbesserten Farbwiedergabe zu erzeugen.

2. Vorrichtung (10; 20) nach dem vorangehenden Anspruch, bei der zumindest entweder
das Beleuchtungslicht einen Farbwiedergabeindex aufweist, der um mindestens 10 höher ist als der Farbwiedergabeindex des Lichts der ersten Lichtquelle (21) oder
der mittlere Farbabstand bei Beleuchtung eines vorbestimmten Satzes von Testfarben mit dem Beleuchtungslicht um mindestens ein Zehntel besser ist als bei Beleuchtung des vorbestimmten Satzes von Testfarben nur mit Licht der ersten Lichtquelle (21) oder
die Summe der Beträge der Abstände der Weißabgleichsparameter von 1 bei Beleuchtung mit dem Beleuchtungslicht geringer ist als bei Beleuchtung mit Licht der ersten Lichtquelle (21).

3. Vorrichtung (10; 20) nach einem der vorangehenden Ansprüche, bei der das von der zweiten Lichtquelle (22) erzeugte Licht innerhalb des Spektrums des von der ersten Lichtquelle erzeugten Lichts liegt.

4. Vorrichtung (10; 20) nach einem der vorangehenden Ansprüche, bei der innerhalb des für das menschliche Auge sichtbaren Spektralbereichs die mittels der Einkopplungseinrichtung (25; 35, 135) in den Strahlengang (27) eingekoppelte Strahlungsleistung höher ist als die Strahlungsleistung des in den Strahlengang (27) einkoppelbaren Lichts der ersten Lichtquelle.

5. Vorrichtung (10; 20) nach einem der vorangehenden Ansprüche, bei der die zweite Lichtquelle zumindest entweder einen Diodenlaser (22) oder einen anderen Laser umfasst.

6. Vorrichtung (10; 20) nach einem der vorangehenden Ansprüche, ferner mit:
einer dritten Lichtquelle (23),
wobei die Einkopplungseinrichtung (25; 35, 135)) ausgebildet ist, um Licht der ersten Lichtquelle (21), Licht der zweiten Lichtquelle (22) und Licht der dritten Lichtquelle (23) in den gemeinsamen Strahlengang (27) einzukoppeln, um das Beleuchtungslicht zu erzeugen,
wobei das Spektrum des Beleuchtungslichts eine bessere Farbwiedergabe aufweist als das erste Spektrum (47) und als eine hinsichtlich der Farbwiedergabe optimale Mischung des Lichts der ersten Lichtquelle (21) und des Lichts der zweiten Lichtquelle (22).

7. Vorrichtung (10; 20) nach dem vorangehenden Anspruch, bei der zumindest entweder
das Beleuchtungslicht einen Farbwiedergabeindex aufweist, der um mindestens 15 höher ist als der Farbwiedergabeindex des ersten Spektrums (47) oder
der mittlere Farbabstand bei Beleuchtung eines vorbestimmten Satzes von Testfarben mit dem Beleuchtungslicht um mindestens ein Fünftel besser ist als bei einer Beleuchtung mit Licht der ersten Lichtquelle (21) oder
die Summe der Beträge der Abstände der Weißabgleichsparameter von 1 bei Beleuchtung mit dem Beleuchtungslicht geringer ist als bei Beleuchtung mit einer hinsichtlich der Farbwiedergabe optimalen Mischung des Lichts der ersten Lichtquelle (21) und des Lichts der zweiten Lichtquelle (22).

8. Vorrichtung (10) nach einem der vorangehenden Ansprüche, ferner mit:
einer Kamera (34) zum Erfassen eines Bilds eines mittels der Vorrichtung (20) beleuchteten Objekts (39).

9. Vorrichtung (10) nach dem vorangehenden Anspruch, bei dem die Farbwiedergabe bei Beleuchtung zu untersuchender Objekte mit dem Beleuchtungslicht und Erfassung mittels der Kamera (34) besser ist als bei Beleuchtung ausschließlich mit dem Licht der ersten Lichtquelle.

10. Vorrichtung (10) nach Anspruch 8 oder 9, ferner mit einem Endoskop (30) oder einem Exoskop oder einem Mikroskop.

11. Verfahren zum Bereitstellen von weißem Beleuchtungslicht für medizinische und boroskopische Anwendungen, mit folgenden Schritten:
Erzeugen (101) von Licht mit einem breiten Spektrum mittels einer ersten Lichtquelle (21), die eine Leuchtdiode umfasst;
Erzeugen (102) von monochromatischem Licht mittels einer zweiten Lichtquelle (22);
Einkoppeln (104) von Licht der ersten Lichtquelle (21) und Licht der zweiten Lichtquelle (22) in einen gemeinsamen Strahlengang (27) mittels einer Einkopplungseinrichtung (25; 35), um Beleuchtungslicht mit einer verbesserten Farbwiedergabe zu erzeugen.

12. Verfahren nach dem vorangehenden Anspruch, bei dem zumindest entweder
das Beleuchtungslicht einen Farbwiedergabeindex aufweist, der um mindestens 10 höher ist als der Farbwiedergabeindex des Lichts der ersten Lichtquelle (21), oder
der mittlere Farbabstand bei Beleuchtung eines vorbestimmten Satzes von Testfarben mit dem Beleuchtungslicht um mindestens ein Zehntel besser ist als bei einer Beleuchtung mit Licht der ersten Lichtquelle (21), oder
die Summe der Beträge der Abstände der Weißabgleichsparameter von 1 bei Beleuchtung mit dem Beleuchtungslicht geringer ist als bei Beleuchtung mit Licht der ersten Lichtquelle (21).
